# EUROPEAN PATENT SPECIFICATION

(11) **EP 3 275 319 B1**
(45) Date of publication and mention of the grant of the patent: **05.08.2020**
(21) Application number: 15885957.9
(22) Date of filing: 23.06.2015
(51) Int. Cl.: A24F 40/30, A24F 13/12, A24F 40/10, A24F 40/20, A24F 40/40, A24F 7/00

(54) **ELECTRIC HEATING TYPE CIGARETTE SMOKING DEVICE HAVING ELECTRONIC CIGARETTE SMOKING FUNCTION**
ZIGARETTENRAUCHVORRICHTUNG MIT ELEKTRONISCHER ZIGARETTENRAUCHFUNKTION
DISPOSITIF DE CIGARETTE DU TYPE À CHAUFFAGE ÉLECTRIQUE AYANT UNE FONCTION DE CIGARETTE ÉLECTRONIQUE

(30) Priority: 23.03.2015 CN 201510127270
(43) Date of publication of application: 31.01.2018
(73) Proprietor: CHINA TOBACCO YUNNAN INDUSTRIAL CO., LTD, Wuhua District Kunming Yunnan 650231 (CN)
(72) Inventor: MIAO, Mingming, Kunming Yunnan 650231 (CN); TANG, Jianguo, Kunming Yunnan 650231 (CN); ZHENG, Xudong, Kunming Yunnan 650231 (CN); SHANG, Shanzhai, Kunming Yunnan 650231 (CN); YUAN, Dalin, Kunming Yunnan 650231 (CN); LEI, Ping, Kunming Yunnan 650231 (CN); SUN, Zhiyong, Kunming Yunnan 650231 (CN); LI, Shoubo, Kunming Yunnan 650231 (CN); CHEN, Yongkuan, Kunming Yunnan 650231 (CN); YANG, Liu, Kunming Yunnan 650231 (CN); YANG, Ji, Kunming Yunnan 650231 (CN); HAN, Yi, Kunming Yunnan 650231 (CN); HAN, Jingmei, Kunming Yunnan 650231 (CN)
(74) Representative: Gille Hrabal
(86) International application number: PCT/CN2015/082078
(87) International publication number: WO 2016/150019

(56) References cited:
- EP-A2- 0 354 661
- CN-A- 103 263 083
- CN-A- 103 271 447
- CN-A- 103 519 351
- CN-U- 202 085 723
- CN-U- 203 538 384
- CN-U- 204 483 008
- JP-A- H05 184 675
- US-A- 4 907 606
- US-A1- 2007 074 734

## Description

### TECHNICAL FIELD OF THE INVENTION

The present invention relates to the field of a traditional electrically-heating cigarette technique, and more particularly to an electrically-heating cigarette smoking device having an electronic cigarette smoking function.

### TECHNICAL BACKGROUND OF THE INVENTION

As the expansion and supplement of a traditional cigarette, the development of a new tobacco product is inevitable. The related research work of an electrically-heating cigarette, an electronic cigarette and so on is also kicked off. The electrically-heating cigarette smokes the tobacco product by heating rather than burning, compared with the traditional cigarette, on the one hand, the technique may effectively reduce the decomposition of harmful ingredients of the tobacco in the high temperature environment, on the other hand, the technique may reduce or even eliminate the side stream smoke of the tobacco product in the process of the use. The electronic cigarette produces the smoke in the way of heating liquid smoke, which has the advantages of environmental protection and no secondhand smoke.

The comfort of the cigarette has a greater relationship with the moisture of the smoke, the study found that the suitability of the smoke moisture would make the smoke be soft and delicate, small irritation, and better sensory comfort; when the smoke moisture content is low, this will lead to dry smoke, bigger irritation and reduced sensory comfort. The nicotine in the cigarette smoke comprises the nicotine under a combination state and the nicotine under a free state, the smoke irritation is mainly determined by the content of the nicotine under the free state. The relative content of the two states of the nicotine is related to the environment (for example, humidity, and PH) of the smoke. The nicotine under the free state may be combined with hydrogen ions in the smoke to form a salt (the nicotine under the combination state), when the moisture content (humidity) of the smoke is low, the hydrogen ion concentration decreases, this will inhibit the formation of the nicotine under the combination state while also decreasing the water solubility of the nicotine under the combination state, this makes it easier to decompose the nicotine under the combination state into the nicotine under the free state, so that the relative content of the nicotine under the free state increases, and the nicotine under the free state/the total nicotine also increases, resulting in the increase in the irritation and dryness. However, if the humidity of the smoke is too large, the nicotine under the free state will be too low, which will fail to reach the physiological strength required by the smoker. Therefore, the change of the moisture content (humidity) of the smoke will significantly affect the state distribution of the nicotine, so that the nicotine under the free state and the ratio of the nicotine under the free state/the nicotine will change significantly. Under low humidity conditions, the content of the nicotine under the free state and the ratio of the nicotine under the free state to the nicotine both increase, so that the cigarette product is easier to produce irritation when being smoked in an dry environment than being smoked in an wet environment. In order to make the product have the higher ratio of the nicotine under the free state to the nicotine, and ensure the stability of the nicotine under different wet environments to solve the problem of the bigger irritation of the product in the dry environment, a glycerol may be added into the tobacco shred to increase the moisture of the cigarette smoke and improve the sensor comfort of the cigarette. However, the study has shown that the glycerol and other humectants in the cigarette shred may have thermal cracking under the burning temperature (900°C) of the cigarette to produce harmful aldehydes (especially acrolein).

When the electrically-heating cigarette is smoked, although the cigarette eliminates the harmful ingredients released by the decomposition under a high temperature environment relative to the traditional cigarette and has a certain tobacco flavor, this also has lowered physiological satisfaction, decreased smoke amount, worse comfort and other problems at the same time. Currently, the problems of dilution and faint smokes due to the lowered tar amount of the cigarette are solved by means of adding the essence and the flavor, to improve the odor of the electrically-heating cigarette, enrich the aroma amount and maintain a good sensory quality. A compensation method in the prior art for adding the essence and the flavor is to add a cotton thread impregnated with liquid flavor essence or to add a flavor microcapsule to the filter tip during the course of the acetate fiber molding of the filter tip so that fragrance is released during the smoking to improve the smoking taste of the electrically-heating cigarette. In the process of the smoking, as the essence and the flavor are increasingly reduced due to the continuous release, the mixture of each smoke and the essence components is inconsistent, so that the smoking taste of each puff of the electrically-heating cigarette is quite different. In addition, the microcapsule containing the flavor may release the aroma components only when being broken, which brings inconvenience to the smoking.

The smoking principle of electric cigarette is as follows: Smoke oil is heated on an electric heating element of an vaporizing system, vaporized into high temperature vapor and is discharged to an opening end. After the discharged vapor is expanded in the atmosphere and condensed into smoke-like tiny liquid droplets, thereby being similar to the traditional cigarette smoke. Unlike traditional cigarette smoke, the aerosol particles of the electronic cigarette smoke are tiny liquid droplets with a smaller particle diameter than that of the aerosol particles in the traditional cigarette smoke.

In terms of sensory quality, compared to the traditional cigarette, the electronic cigarette generally has the shortcoming that the saturation of the smoke in the mouth is insufficient, the so-called saturation refers to the impact or the sense of presence felt by the smoker in his mouth in the process of the smoking. The sound saturation means that the smoker feels the obvious presence of the smoke during the smoking process and does not have empty feeling. In addition, according to the electronic cigarette smoker, the nicotine-containing smoke oil of the electronic cigarette in the prior art has heavier laryngeal and nasal irritation. Although the tobacco flavor or tobacco extraction liquid may be added to the smoke oil of the electronic cigarette, its tobacco flavor is still not obvious enough.

The above reasons lead to that the consumer of the electrically-heating cigarette or electronic cigarette does not have ideal smoking sensory experience, therefore, the electrically-heating cigarette or the electronic cigarette does not have high market acceptance.

CN203538384U discloses a kind of electronic cigarette holder with two taste one functions, comprises the electronic cigarette body with atomizer assembly, the front end of electronic cigarette body is provided with cutting ferrule, the front end of cutting ferrule is provided with jack of placing cigarette for inserting, the middle part of cutting ferrule is provided with through hole, and jack is communicated with the atomizer assembly of electronic cigarette holder by through hole. The utility model has the function of electronic cigarette and cigarette holder concurrently, both can as electronic cigarette, use separately, can be used as again cigarette holder mixes the mixture of sucking relieve sore throat clearing lung-heat class plant medical herbs, tobacco or above-mentioned substance and realizes two taste one on the basis of sucking electronic cigarette tobacco tar, can synergy cigarette suck impression, the auxiliary harmful components that reduce in tobacco, reduce and alleviate stimulation and the bad impression of tobacco to respiratory tract, the auxiliary degree of dependence of smoker to cigarette that reduce, reduces smoker craving for tobacco gradually.

In view of the above-mentioned problems, the present invention provides an electrically-heating cigarette smoking device having an electronic cigarette smoking function which is capable of sufficiently combining the advantages of the electrically-heating cigarette and the electronic cigarette to provide a better cigarette smoking sensory experience.

### SUMMARY OF THE PRESENT INVENTION

In view of the above deficiencies of the prior art, the present invention provides an electrically-heating cigarette smoking device having an electronic smoking function, the present invention aims at increasing the smoke amount of the electrically-heating cigarette by mixing the mainstream smoke of the cigarette and vaporized vapor of the electronic cigarette, improving the smoking sensory quality, reducing the harm of the smoking to the human body and the surrounding environment, and changing the compensation technique in the prior art of the essence and the flavor to increase the consistency of smoking taste of each puff of the electrically-heating cigarette and expand the use function of the electrically-heating cigarette. The object of the invention is obtained through the following technical solution:
The present invention relates to an electrically-heating cigarette smoking device having an electronic smoking function, comprising an electrically-heating cigarette holder 1 having an electric heater, an electronically-vaporizing component and a mouth piece 15 which are mutually matched for assembly; wherein the electrically-heating cigarette holder 1 comprises an electrically-heating cigarette holding cavity and an optional end cap; the electronically-vaporizing component comprises a shell 7 and the following assemblies : a power supply 5, an air flow sensor switch 3 and an electric vaporizer 8 provided with a central air flow passage, located in the shell 7; the mouth piece 15 has an air flow passage 16; the optional end cap is used for closing one open end of the shell 7 when in existence; the electrically-heating cigarette smoking device also comprises an air flow sensor switch gas inlet 2 perforating the shell 7 and/or the optional end cap of the electrically-heating cigarette holder, the air flow sensor switch gas inlet 2 is in air communication with the air flow passage 16 of the mouth piece 15 via the air flow sensor switch 3; the electronic vaporizer 8 and the electrically-heating cigarette holder 1 are arranged in parallel; the central air flow passage of the electronic vaporizer 8 and the electrically-heating cigarette holding cavity 1 are both in air communication with the air flow passage 16 in the mouth piece 15.

The air flow sensor switch gas inlet 2 perforating the shell 7 and the end cap of the electrically-heating cigarette holder is required to ensure that intake air flow may quickly trigger the air flow sensor switch 3. When in use, the electrically-heating cigarette is inserted into the electrically-heating cigarette holding cavity in the electrically-heating cigarette holder; as the electric vaporizer will form a negative pressure inside the electronically-vaporizing component due to the smoking actions of the smoker, the ambient air enters into the inner part of the electronically-vaporizing component through the air flow sensor switch gas inlet 2 and triggers the air flow sensor switch 3. At the same time, since the air flow sensor switch 3 triggers so that the power supply 5 and the electronic vaporizer 8, as well as the power supply 5 and the electric heater are turned on through a connection terminal respectively, the substance in the electronic vaporizer is heated to generate vaporized vapor, the formed vaporized vapor enters into the central air flow passage of the electronic vaporizer; the electric heater begins to heat, and the electrically-heating cigarette is heated to generate mainstream smoke. The mainstream smoke generated by the heated electrically-heating cigarette and the vaporized vapor generated by the electronic vaporizer 8 are smoked into the air flow passage 16 in the mouth piece 15 or are first mixed before entering the air flow passage 16 in the mouth piece 15 and then enter the air flow passage 16 in the mouth piece 15; at this time, the ambient air may enter the air flow passage 16 in the mouth piece 15 for smoking by the smoker before being mixed with the mainstream smoke or with the vaporized vapor generated by the electronic vaporizer 8, or with the above two. The mainstream smoke refers to the smoke smoked from a smoking end of the cigarette rod when the cigarette is smoked, i.e., the portion capable of being smoked from the generated smoke, the role of the ambient air is to dilute the mainstream smoke as a dilution air and to cool the mainstream smoke.

The connection terminal connects the power supply 5 to the electronic vaporizer 8 and the electric heater or is connected with the power supply 5 or an external charging device. The power supply 5 is a disposable battery or a rechargeable battery. Preferably, the rechargeable battery is a polymer lithium battery. The electronic vaporizer may be any electronic vaporizer in the prior art, the operating temperature of the electronic vaporizer is 150 °C to 350 °C.

In a preferred embodiment, the electric heater is arranged in a way of circumferential heating, central heating, bottom heating or the combination thereof, the electrically-heating cigarette holding cavity is used for holding the special electrically-heating cigarette with the circumference of 24-26mm and the length of 35-75mm, and can heat the cigarette to 220-350°C within 20 seconds.

In a preferred embodiment, part or all of the air flow passage 16 within the mouth piece 10 is a helical air flow passage 17 which comprises a connection end 18 at the downstream thereof and a suction end 19 at the upstream thereof.

In a preferred embodiment, the helical air flow passage 17 has an equal diameter or a varying diameter.

In a preferred embodiment, the diameter of the air flow passage of the helical air flow passage 17 is always equal from the connection end 18 to the suction end 19, gradually changed in a continuous gradient from the big to the small or gradually changed in a continuous gradient from the small to the big.

In a preferred embodiment, the shell 7 is divided into two cavities, the first cavity receives the electronic vaporizer 8 while the second cavity receives the power supply 5 and the air flow sensor switch 3. The two cavities may be relatively self-enclosed, but may have a necessary gas inlet hole. Or, the two cavities may have an open end and is relatively closed by the cooperation between the electrically-heating cigarette holding cavity of the electrically-heating cigarette holder and the end cap.

In a preferred embodiment, the vaporizer 8 comprises an electric heating wire, a liquid storing cavity for storing a liquid to be vaporized, and a liquid guiding device for guiding the liquid to be vaporized to the electric heating wire.

In a preferred embodiment, the electric heating wire is located in the central air flow passage; independently, the liquid guiding device is a liquid guiding rope or a liquid guiding pipe.

Preferably, the liquid to be vaporized contains one or more of a flavoring substance, a drug extract or a glycerol. The liquid to be vaporized is a liquid containing a volatile substance and capable of being vaporized upon heating. The liquid to be vaporized evaporates and condenses into an aerosol, which is smoked by the smoker after being mixed with the mainstream smoke of the electrically-heating cigarette and the dilution air. When the mainstream smoke of the electrically-heating cigarette pass the wet environment containing the volatile material, the volatile material facilitates the migration of the nicotine to the smoke and releases the free (aprotic) nicotine produced by the vaporization of the smoke particles, making the free nicotine migrate in the form of vapor, being conducive to increasing the amount of the free nicotine in the smoke, thereby enhancing the physiology sensory strength of the smoker. The different compositions of the liquid to be vaporized may also play different roles in the mainstream smoke of the electrically-heating cigarette, including the action of reducing the damage of the mainstream smoke of the electrically-heating cigarette, the action of improving the smoking sensory quality of the electrically-heating cigarette, and the action of supplementing the aroma composition of the mainstream smoke of the electrically-heating cigarette or an auxiliary pharmacological action. Preferably, the liquid to be vaporized is the glycerol having hygroscopicity or a mixed liquid taking the glycerol as a main component and containing the volatile substance. Due to the hygroscopicity thereof, the glycerol may increase the moisture content of the mainstream smoke, thereby reducing the irritation of the mainstream smoke, improving the delicate softness of the smoke, and significantly improving the sensitivity of the electrically-heating cigarette.

Preferably, the volatile substance may be the flavoring substance or other functional components, the flavoring substance may be the essence and the flavor, which, after evaporation, are mixed with the mainstream smoke of the electrically-heating cigarette, this serves to supplement the aroma while ensuring the consistency of the taste of each smoking; the other functional components include some pharmacologically active drug extracts which, after evaporation, are mixed with the mainstream smoke of the electrically-heating cigarettes, this functions as a certain therapeutic effect while satisfying the physiological feelings of the smoking.

In a preferred embodiment, the electrically-heating cigarette smoking device having the electronic smoking function further comprises a light transmission hole 6 of a status indicator lamp located on the shell 7 and a status indicator lamp (not shown) located in the shell 7. Preferably, the electrically-heating cigarette smoking device further comprises a light guide base 9 located on the light transmission hole 6 of the status indicator lamp, the light guide base 9 is actually one closing cap of the light transmission hole 6 of the indicator lamp, the light of the status indicator light is emitted through the light transmission hole 6 of the status indicator lamp and the light guide base 9. The light guide base 9 may prevent dust from falling into the inside of the shell 7 through the light transmission hole 6 of the status indicator lamp. When the power supply is operated, the light of the status indicator lamp connected to the power supply 5 may transmits out from the light guide base 9 and the light transmission hole 6 of the status indicator lamp 6 to indicate the operating state of the electrically-heating cigarette smoking device.

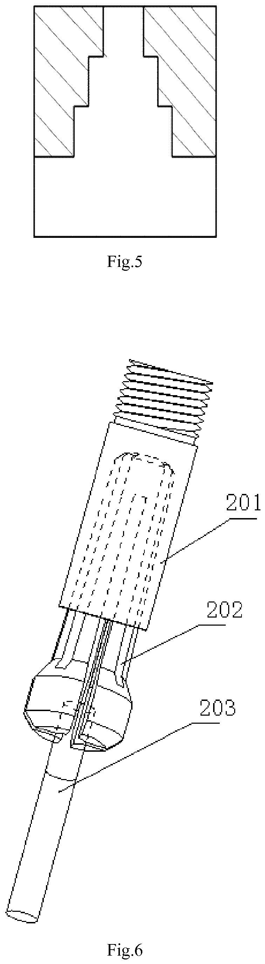

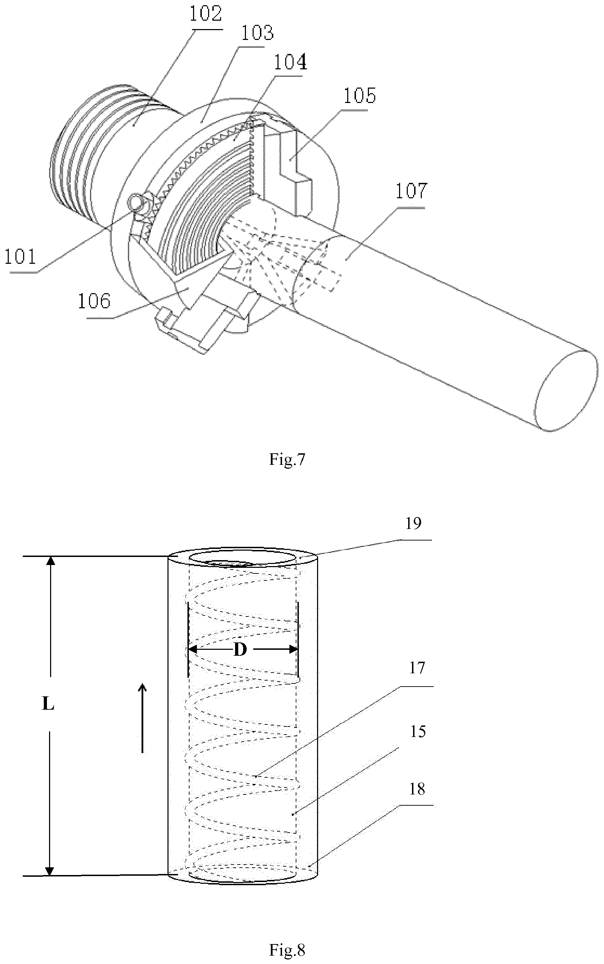

The electrically-heating cigarette holder 1 may be any holder capable of being connected to the electronically-vaporizing component of the present invention and capable of holding the electrically-heating cigarette in the prior art. The electrically-heating cigarette holder 1 and the electronically-vaporizing component are fixedly connected or detachably connected. The electrically-heating cigarette holder may optionally have an end cap that may be used for enclosing an open end of the shell 7 of the electronically-vaporizing component. At this time, this is equivalent to the end cap and the shell 7 together to form a relatively enclosed space. The electrically-heating cigarette holder may also have no end cap, in which case the shell 7 is required to be relatively self-enclosed and does not have the open end, but the necessary gas inlet hole is provided on the end surface or side surface thereof. Preferably, the electrically-heating cigarette holding cavity in the electrically-heating cigarette holder 1 may meet the need for airtight bonding of the electrically-heating cigarette with different circumferences or a electrically-heating cigarette filter tip by providing different cigarette holding devices therein. In a preferred embodiment, the electrically-heating cigarette holding cavity of the electrically-heating cigarette holder 1 is provided therein with a variable-diameter holding device to hold the electrically-heating cigarettes with variable diameters, the variable-diameter holding device is selected from a plurality of overlapping washers which are successively reduced in diameter from an opening of the electrically-heating cigarette holding cavity, or a spring clamping head, or a multiple-jaw chuck holder. For a method in the prior art for regulating the holding diameter of the electrically-heating cigarette holder, the holder is provided therein with the washers with different inner diameters, the washers are overlapped in turn from the large to the small, to hold the electrically-heating cigarettes with different diameters, as shown in Fig. 5. For another method in the prior art for regulating the holding diameter of the holder, the spring clamping head is used, as shown in Fig. 6, the spring clamping head is a plurality of spring clamping pieces 202 which are arranged in a sleeve 201 provided with a threaded joint, are provided in a conical arrangement and are co-end, after being inserted with a holding object 203, the spring clamping piece is extruded and deformed, the holding diameter thereof is regulated by means of the elasticity of the spring clamping piece itself. Another industry-common holder with an adjustable holding diameter is the multiple-jaw chuck holder, among them, the most common one is a three-jaw chuck holder, shown in Fig. 7 and is the prior art, The specific structure and operating principle thereof may be summarized as follows: the three-jaw chuck holder has a holder mounting screw 102 which is provided thereon with a thread for mounting on other apparatuses. the mounting screw 102 is provided thereon with a holding block support 103 and a holding block adjusting disk which are coaxially arranged therewith and have flange structures, one side of the holding block Adjusting Disk 104 is provided with a bevel gear, and the other side thereof is provided with teeth which are a plane thread (a vortex line) shape (for example, a coiled mosquito-like shape) along an axis center, three movable holding blocks 105 are meshed with the teeth through a tooth space itself, the three movable holding blocks 105 are used for holding a held object 107. One adjusting gear 101 is located at the edge of the holding block Adjusting Disk and meshed with the bevel gear on the holding block Adjusting Disk 104 through the bevel gear itself. The three movable holding blocks 105 are each limited by two movable block stoppers fixed on the holding block support 103 so that the movable holding block 105 may only move radially without rotation, In Fig. 7, only two sets of the movable block stoppers are shown in order to simplify the drawing. When a user manually rotates the Adjusting Gear 101, the Adjusting Gear 101 rotates the holding block Adjusting Disk 104 through the meshing action of the bevel tooth, and further rotates the teeth thereon which are the plane thread (the vortex line) shape, by means of the meshing relationship between the tooth spaces between these teeth and the movable holding block 105, the rotation movement of the teeth along the axis center is converted into the radial movement of the movable block close to or away from the axis center, so as to realize the continuous adjustment of a holding diameter. More details on the multiple-jaw chuck holder may be found in relevant technical data in the prior art and are not repeated in this paper again. In addition, the present inventor also invents another novel drawbar electrically-heating cigarette holder, which may also continuously adjust the holding diameter and will be applied in another application along with the relevant content of the drawbar holder.

In a preferred embodiment, the air flow sensor switch gas inlet 2 is located on the side surface or the end surface of the portion of the shell surrounding the second cavity.

In a preferred embodiment, the air flow sensor switch 3, the electronic vaporizer 8 and the electric heater are all electrically connected to the power supply 5, an electric connection way therebetween enables the air flow sensor switch 3 to control whether the electronic vaporizer 8 and the electric heater are electrified or not. Preferably, a silicone sleeve 4 is provided on the periphery of the air flow sensor switch 3. The silicone sleeve 4 is used for airtightly fixing the air flow sensor switch 3 so that the air flow entering from the air flow sensor switch gas inlet 2 completely pass through the air flow sensor switch 3 to effectively trigger the air flow sensor switch 3. The air flow sensor switch 3 and the silicone sleeve 4 together constitute an air flow sensor.

Preferably, the shell 7 also comprises the connection terminal therein, the connection terminal comprises a positive electrode 12 and a negative wire connection base 10; the positive electrode 12 is connected to the power supply 5; the negative wire connection base 10 is connected to the electronic vaporizer 8 or to an external charging device. When the electrically-heating cigarette is smoked, the connection terminal serves to connect the electronic vaporizer and the power supply, the positive electrode 12 of the connection terminal is connected to the power supply 5, the negative wire connection base 10 is connected to the electronic vaporizer 8; when the power supply 5 is a chargeable power supply and needs to be charged, the connection terminal functions as the external charging device and the power supply, the positive electrode 12 of the connection terminal is connected to the power supply 5, the negative wire connection base 10 is connected to the external charging device, the power supply 5 is charged.

In a preferred embodiment, the connection terminal also comprises an insulating sleeve 11 located between the positive electrode 12 and the negative wire connection base 10. The insulating sleeve 11 is used for blocking the negative wire connection base 10 and the positive electrode 12.

The second aspect of the present invention relates to a use of the electrically-heating cigarette smoking device having an electronic cigarette smoking function described in the first aspect of present invention, it is used for increasing smoke saturation and/or reducing laryngeal or nasal irritation for a smoker by nicotine in a smoke.

The present invention has the following beneficial effects:
1. An electrically-heating cigarette smoking device having an electronic cigarette smoking function according to the present invention is practically a smoking device combining an electrically-heating cigarette and an electronic cigarette, wherein the electrically-heating cigarette and the vaporizer can be used simultaneously or selected uniquely for use, one device can provide three smoking ways of the electrically-heating cigarette, the electronic cigarette or a combination of the above two.
2. The electrically-heating cigarette smoking device having an electronic cigarette smoking function in the present invention adopts the spiral air flow passage design to solve the problem of the poor saturation of the smoke, and also greatly reduce the laryngeal and nasal irritation for the smoker by the nicotine in the smoke oil.

The electrically-heating cigarette smoking device having an electronic cigarette smoking function of the present invention has the following advantages in comparison with the electrically-heating cigarette smoking method in the prior art:
1. Compared to the electrically-heating cigarette smoking way in the prior art, the electrically-heating cigarette is smoked through the electrically-heating cigarette smoking device having the electronic smoking function according to the present invention, this dilutes the mainstream smoke of the electrically-heating cigarette and reduces main harmful components while further reducing piquant and spicy feeling and improving smoking sensory quality, this is because the glycerol and other substances in the electronic vaporizer may increase the moisture content of the mainstream smoke due to the hygroscopicity, thereby reducing the irritation of the mainstream smoke, improving the delicate softness of the smoke and significantly improving the sensory comfort of the electrically-heating cigarette.
2. When the mainstream smoke of the electrically-heating cigarette pass through the air flow passage containing the vaporized vapor in the electronic vaporizer, the vaporized vapor and the wet environment facilitate the migration of the nicotine to the smoke and releases the free (aprotic) nicotine produced by the vaporization of the smoke particles, making the free nicotine migrate in the form of vapor, being conducive to increasing the amount of the free nicotine in the smoke, thereby enhancing the physical sensory strength of the smoker.
3. In the electrically-heating cigarette smoking device of the present invention, the glycerol is evaporated at low temperature, reducing the content of aldehydes (especially acrolein) produced by the pyrolysis of the glycerol; the glycerol-containing smoke particles increase the particle size due to the easier absorption of water steam, so that the remained smoke particle-phase matter increases, therefore, more smoke particles remain in the mouth, reducing the particle-phase matter in the mainstream smoke remained in the lungs, and reducing the damage of the smoking to the lungs.
4. Through the vaporization of the liquid to be vaporized with different functions, different smoking feelings can be brought for the electrically-heating cigarette, compared to the way that function components are added to the filter tip in the prior art, the smoking device is used more conveniently; furthermore, the ways including the way of adding the drug extract to the liquid to be vaporized also may function as a therapeutic purpose, not only reducing the damage of the smoking but also being capable of functioning as the therapeutic purpose.
5. As the filter tip of the cigarette is airtightly inserted into the electrically-heating cigarette holder, the inhaled air is mainly from the diluted air entering from the shell of the electronically-vaporizing component or end cap of the electrically-heating cigarette holder, correspondingly reducing the air amount entering the cigarette from the end of the electrically-heating cigarette, slowing down the burning speed of the electrically-heating cigarette, reducing the intake amount of the harmful substances of each smoking and adding the number of the smoking.
6. Compared to the smoking method of the electrically-heating cigarette in the prior art, when smoking the cigarette, the electrically-heating cigarette smoking device having an electronic cigarette smoking function of the present invention can sufficiently utilizes the advantage of the large amount of the smoke after the vaporization of the electronic cigarette and make up for the problem of being lack of smoke amount in electrically-heating cigarette.
Compared to the electronic cigarette containing nicotine in the smoke oil in the prior art, the electrically-heating cigarette smoking device having the electronic smoking function has the following advantages:
1. For the electronic cigarette containing the tobacco taste in the smoke oil in the prior art, the smoke oil containing the glycerol as the main component contains a certain concentration of the tobacco extraction liquid and is delivered together with the vaporized smoke oil, however, for the electrically-heating cigarette smoking device having an electronic cigarette smoking function of the present invention, the electrically-heating cigarette is firstly heated and releases the tobacco intrinsic flavor which is mixed with the vaporized vapor in the vaporizer and delivered. In comparison with the two cases, the tobacco intrinsic flavor delivered by the electrically-heating cigarette smoking device of the present invention originates from the tobacco leaf itself, is endogenous and is released by the heating of the electrically-heating cigarette; however, the tobacco extraction liquid delivered by the electronic cigarette having the tobacco taste in the prior art is manually added into the smoke oil, is exogenous and is released through the evaporation of the smoke oil. The tobacco extraction liquid used by the latter is mainly from a tobacco extract, having a complex extraction process and higher cost, furthermore, the heating temperature of the electronic cigarette in the prior art is lower than the heating temperature of the electrically-heating cigarette, and the released amount of the tobacco intrinsic flavor is not enough, however, the device of the present invention may ensure the release of the tobacco intrinsic flavor while reducing the damage of other harmful substances to the health.
2. When the electronic cigarette in the prior art is smoked, the delivery efficiency of the nicotine of the electronic cigarette in the prior art is lower than that of the traditional electrically-heating cigarette. The nicotine delivered by the electrically-heating cigarette smoking device of the present invention is actually from the traditional electrically-heating cigarette, and therefore, the nicotine delivery efficiency is higher than that of the electronic cigarette in the prior art.
3. The smoke oil of the electronic cigarette containing the nicotine in the prior art is often added with the flavoring substances, such as mint, vanilla, fruit flavor substances and so on. When the nicotine is dissolved in the smoke oil containing these flavoring substances, the nicotine is easily oxidized by these flavoring substances, affecting the stability of a product. However, for the electrically-heating cigarette smoking device of the present invention, the nicotine and the favoring substances in the liquid to be vaporized may be mixed as the form of the aerosol only when being smoked, due to the short mixing time and the low content of each smoking, there is no the problem of nicotine deterioration in the nicotine-containing electronic cigarette in the prior art.
4. Compared to the nicotine-containing electronic cigarette in the prior art, the electrically-heating cigarette smoking device in the present invention significantly increases the content of the nicotine in the inhaled smoke in the same volume, having better physiological satisfaction of the nicotine and significantly reducing the smoking sweet feeling of the electronic cigarette in the prior art (glycerol and propylene glycol as the main components of a smoke oil solvent).

### BRIEF DESCRIPTION OF THE DRAWINGS

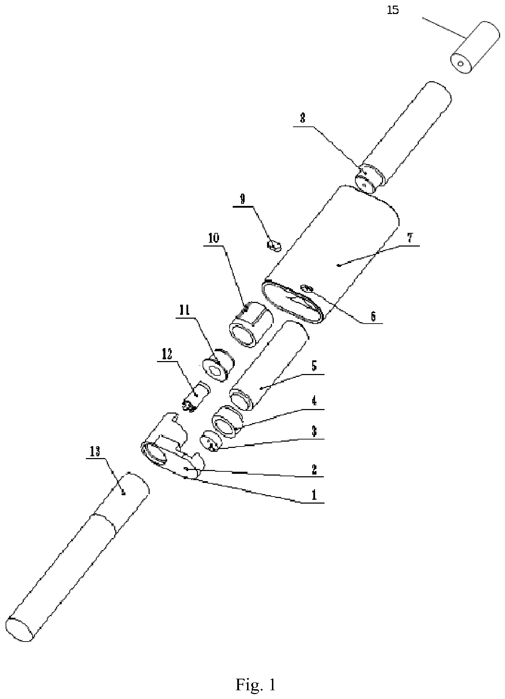

Fig. 1 is exploded views of various components of the electrically-heating cigarette smoking device having an electronic smoking function.

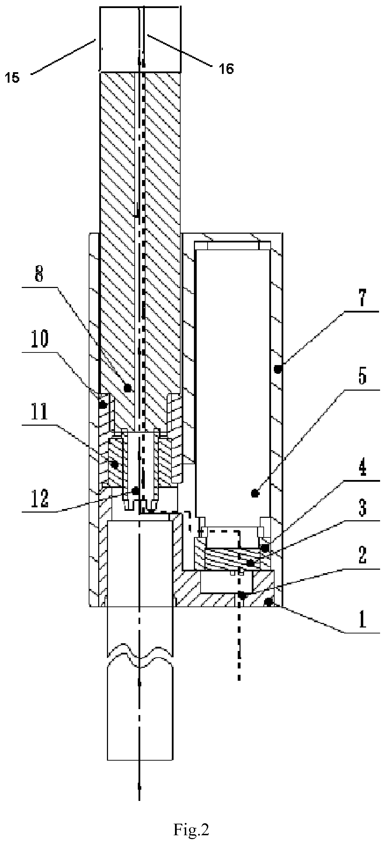

Fig. 2 is an internal cross-sectional view of an electrically-heating cigarette smoking device. An air flow sensor switch gas inlet 2 is located on an end cap of the electrically-heating cigarette holder 1. A dotted line indicates the mainstream smoke of the electrically-heating cigarette; a dashed line indicates the diluted air flow inhaled after an air flow sensor switch is triggered. The mainstream smoke of the electrically-heating cigarette and the diluted air flow entered an electronic vaporizer and are mixed with a vaporized vapor in the electronic vaporizer, and then are inhaled by the smoker.
Fig. 3 is an internal cross-sectional view of an electrically-heating cigarette smoking device. air flow sensor switch gas inlet 2 is located on a shell of an electronically-vaporizing component. A dotted line indicates the mainstream smoke of the electrically-heating cigarette and a dashed line indicates the diluted air flow inhaled after an air flow sensor switch is triggered. The mainstream smoke of the electrically-heating cigarette and the diluted airflow are inhaled after entering an electronic vaporizer and being mixed with an vaporized vapor in the electronic vaporizer.

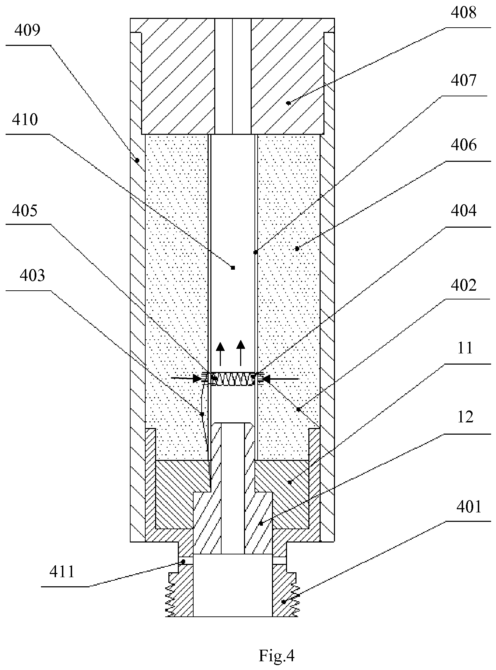

Fig. 4 is a structural view of an electronic vaporizer 8 used in the device of figure 2. direction of an arrow indicates the direction of the delivery of the liquid to be vaporized and the vaporized vapor. The liquid to be vaporized is guided from an oil storage cotton 406 to a heating wire 405 through a glass fiber oil-guide rope 404, is heated and vaporized on the heating wire, forms a vapor in an air flow passage, and then is mixed with the mainstream smoke of an electrically-heating cigarette entered from the bottom of the air flow passage and the inhaled diluted air flow after an air flow sensor switch is triggered.
Fig. 5 is a structural view of an electrically-heating cigarette holder in the prior art that changes a diameter via a plurality of overlapped washers.
Fig. 6 is a view of an electrically-heating cigarette holder in the prior art that clamps and holds an electrically-heating cigarette with a spring clamping head.
Fig. 7 is a view of a multiple-jaw chuck type variable diameter electrically-heating cigarette holder in the prior art for holding an electrically-heating cigarette.
Figure 8 is a view of a helical air flow passage in the device of figure 2 , wherein the helical air flow passage is a view of an equal-diameter helical air flow passage, wherein the arrow direction represents the flow of smoke, "L" represents a radial length; "D" represents a helix diameter.
Figure 9 is a view of a helical air flow passage in the device of figure 3, wherein the helical air flow passage is a view of a non-equal diameter helical airflow passage, wherein the arrow direction represents the flow of smoke.
Figure 10 is a partial enlarged view of the distribution of smoke aerosol particles in a helical air flow passage in an electrically-heating cigarette smoking device, wherein "d" represents the diameter of the air flow passage.

Each reference sign indicates the following meanings:
1-Electrically-heating cigarette holder; 2-Air flow sensor switch Gas inlet; 3-Air flow sensor switch; 4-Silicone Sleeve; 5- Power Supply; 6-Light Transmission Hole of Status Indicator Lamp; 7-Shell; 8-Electronic vaporizer; 9 - Light Guide Base; 10 - Negative Wire Connection Base; 11 - Insulating Sleeve; 12 - Positive Electrode; 13 - Cigarette; 14 -Side Cap of Shell; 15- mouth piece; 16- air flow passage; 17- helical air flow passage; 18-connection end; 19-smoking end; 20-inside of the air flow passage inside. 21-outside of the air flow passage.
401 - Threaded Riveted Joint; 402 - Negative Wire; 403- Positive Wire; 404-Glass Fiber Oil-Guide Rope; 405 - Heating Wire; 406 - Oil Storage Cotton; 407 - Fiber Sleeve; 408 - Top End Cap; 409 - Housing ; 410 - Air flow Passage; 411 -Gas inlet hole;
201 - Sleeve; 202 - Spring Clamping Piece; 203- Held Object;
101 - Adjusting Gear; 102 - Mounting Screw; 103- Holding Block Support; 104- Holding Block Adjusting Disk; 105 - Movable Holding Block; 106 - Movable Block Limit; 107 - Held Object.

### EXAMPLES

### Example 1

An electrically-heating cigarette smoking device having an electronic smoking function as shown in Fig. 2 comprises an electrically-heating cigarette holder 1, an electronically-vaporizing component and a mouth piece which are connected with one another; wherein the electrically-heating cigarette holder 1 comprises an electrically-heating cigarette holding cavity and an end cap which are arranged in parallel; the electronically-vaporizing component comprises a shell 7 and the following assemblies located in the shell 7: a power supply 5 (a rechargeable polymer lithium battery), an air flow sensor switch 3, a connection terminal and an electronic vaporizer 8 provided therein with a central air flow passage; the mouth piece 15 is provided with an airflow passage 16; the electrically-heating cigarette smoking device also comprises an airflow sensor switch gas inlet 2 perforating the shell 7 and the optional end cap of the electrically-heating cigarette holder, the air flow sensor switch gas inlet 2 is in air communication with the air flow passage of the mouth piece 15 through the air flow sensor switch 3; the electronic vaporizer 8 and the electrically-heating cigarette holder 1 are arranged in series. The electrically-heating cigarette holder 1 and the electronically-vaporizing component are fixedly connected.

The shell 7 is divided into two cavities, the first cavity receives the electronic vaporizer 8 and the connection terminal while the second cavity receives the power supply 5 and the air flow sensor switch 3.

The air flow sensor switch gas inlet 2 perforates the end cap of the electric type cigarette holder. The direction of the intake airflow of the air flow sensor switch gas inlet 2 is parallel to the insertion direction of a cigarette rod. The air flow sensor switch gas inlet 2 perforating the end cap of the electrically-heating cigarette holder may ensure that the air flow may quickly trigger the air flow sensor switch 3. When in use, the electrically-heating cigarette is inserted into the electrically-heating cigarette holding cavity in the electrically-heating cigarette holder; as the electric vaporizer will form a negative pressure inside the electronically-vaporizing component due to the smoking actions of the smoker, the ambient air enters into the inner part of the electronically-vaporizing component through the air flow sensor switch gas inlet 2 and triggers the air flow sensor switch 3. At the same time, since the air flow sensor switch 3 triggers so that the power supply 5 and the electronic vaporizer 8, as well as the power supply 5 and the electric heater are turned on through a connection terminal, respectively, the substance in the electronic vaporizer is heated to generate vaporized vapor, the formed vaporized vapor enters into the central air flow passage of the electronic vaporizer; the electric heater begins to heat, and the electrically-heating cigarette is heated to generate mainstream smoke. Since the electronic vaporizer 8 and the electrically-heating cigarette holder 1 are arranged in series, the ambient air entering the interior of the electronically-vaporizing component enters the central air flow passage of the electronic vaporizer; At the same time, the mainstream smoke generated by the heated electrically-heating cigarette is sucked into the central air flow passage of the electronic vaporizer, mixed with the ambient air in the electronic vaporizer, or mixed with the ambient air before entering into the central air flow passage of the electronic vaporizer, the air flow then enters the air flow passage 16 in the mouth piece 15 for smoking by the smoker. The mainstream smoke refers to the smoke smoked from a smoking end of the cigarette rod when the cigarette is smoked, i.e., the portion capable of being smoked from the generated smoke. The role of the ambient air is to dilute the mainstream smoke as a dilution air and to cool the mainstream smoke.

The specific structure of the electronic vaporizer 8 shown in Fig. 4 is a disposable electronic vaporizer and comprises an electric heating wire connected with the connection terminal, a liquid storing cavity for storing an liquid to be vaporized, and a liquid guiding device for guiding the liquid to be vaporized to the electric heating wire. The electric heating wire of the electronic vaporizer 8 is located in the central air flow passage; the liquid guiding device is a liquid guiding rope; the downstream of the center air flow passage of the electronic vaporizer 8 is connected to a mouthpiece.

The electrically-heating cigarette smoking device having the electronic smoking function of Example 1 further comprises a light transmission hole 6 of a status indicator lamp located on the shell 7 and a status indicator lamp (not shown) located in the shell 7. The electrically-heating cigarette smoking device further comprises a light guide base 9 located on the light transmission hole 6 of the status indicator lamp. When the power supply is operated, the light of the status indicator lamp connected to the power supply 5 may be transmitted out from the light guide base 9 to indicate the operation state of the electrically-heating cigarette smoking device.

The air flow sensor switch 3, the vaporizer 8 and the electric heater are all electrically connected to the power supply 5, an electric connection way therebetween enables the air flow sensor switch 3 to control whether the electronic vaporizer 8 and the electric heater are electrified or not. A silicone sleeve 4 is provided on the periphery of the air flow sensor switch 3. The silicone sleeve 4 is used for airtightly fixing the air flow sensor switch 3 so that the air flow entering from the air flow sensor switch gas inlet 2 completely perforate the air flow sensor switch 3 to effectively trigger the air flow sensor switch 3. The air flow sensor switch 3 and the silicone sleeve 4 together constitute an air flow sensor.

The shell 7 also comprises the connection terminal therein, the connection terminal comprises a positive electrode 12 and a negative wire connection base 10; the positive electrode 12 is connected to the power supply 5; the negative wire connection base 10 is connected to the electronic vaporizer 8. The connection terminal also comprises an insulating sleeve 11 located between the positive electrode 12 and the negative wire connection base 10. The insulating sleeve 11 is used for blocking the negative wire connection base 10 and the positive electrode 12. When the electrically-heating cigarette is smoked, the connection terminal serves to connect the electric vaporizer and the power supply, the positive electrode 12 of the connection terminal is connected to the power supply 5, the negative wire connection base 10 is connected to the electric vaporizer 8; when the power supply 5 needs to be charged, the connection terminal functions as the external charging device and the power supply, the positive electrode 12 of the connection terminal is connected to the power supply 5, the negative wire connection base 10 is connected to the external charging device, the power supply 5 is charged.

### Example 2

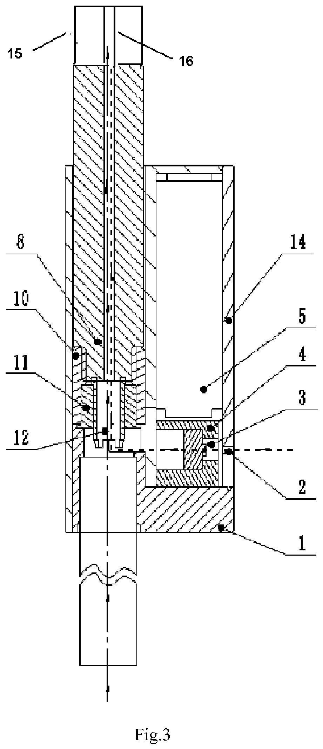

As shown in Fig. 3, an electrically-heating cigarette smoking device having an electronic smoking function differs from the electrically-heating cigarette smoking device of Example 1 in that the electrically-heating cigarette holder 1 and the electronically-vaporizing component in the electrically-heating cigarette smoking device are detachably connected; an air flow sensor switch gas inlet 2 perforates a shell 7 of the electronically-vaporizing component, The direction of the intake air flow of the air flow sensor switch gas inlet 2 is vertical to the insertion direction of a cigarette; the electronic vaporizer 8 has a liquid storage cavity which may be filled with an liquid to be vaporized and comprise an electric heating wire connected to the connection terminal; the liquid to be vaporized comprises glycerol, a flavoring substance and a drug extract. The device according to Example 2 also comprises an side cap 14 of the shell, which can be opened to facilitate the installation of an internal component of the shell.

### Example 3

Example 3 relates to an electrically-heating cigarette smoking device having an electric cigarette smoking function which differs from an electrically-heating cigarette smoking device in Example 1 in that: wherein the electronic vaporizer 8 and the electrically-heating cigarette holder 1 are arranged in parallel. During the smoking process, the mainstream smoke generated by the heated electrically-heating cigarette and the vaporized vapor generated by the electronic vaporizer 8 are smoked into the airflow passage 16 in the mouth piece 15 or are first mixed before entering the airflow passage 16 in the mouth piece 15 and then enter the air flow passage 16 in the mouth piece 15;at this time, the ambient air may enter the air flow passage 16 in the mouth piece 15 for smoking by the smoker after being mixed with the mainstream smoke or with the vaporized vapor generated by the electronic vaporizer 8, or with the above two. The mainstream smoke refers to the smoke smoked from a suction end of the cigarette rod when the cigarette is smoked, i.e., the portion capable of being smoked from the generated smoke. The role of the ambient air is to dilute the mainstream smoke as a dilution air and to cool the mainstream smoke.

The evaluation of the performance of the electrically-heating cigarette smoking device and the comparison with the electrically-heating cigarette smoking devices in the prior art are shown in Table 1 - Table 4.

**Table 1: Comparison of the smoking amount between the device of the present invention and the smoking ways in the prior art of the same cigarette**

| Cigarette sample No. | Smoking ways in the prior art | The present invention |
|---|---|---|
| Index scope | 0-10 | 0-10 |
| 1 | 6 | 8 |
| 2 | 6 | 8 |
| 3 | 7 | 8 |
| 4 | 6 | 9 |
| 5 | 7 | 8 |
| 6 | 8 | 9 |

**Table 2: Comparison of the smoking sensory quality between the device of the present invention and the smoking ways in the prior art of the same cigarette.**

| Cigarette sample No. | 1 | | 3 | | 5 | |
|---|---|---|---|---|---|---|
| Index scope | 0∼10 | | 0∼10 | | 0∼10 | |
| Smoking ways | The prior art | The present invention | The prior art | The present invention | The prior art | The present invention |
| Comfort characteristics | 0∼10 | | 0∼10 | | 0∼10 | |
| Oral irritation / tongue burning | **7** | **8** | **8** | **9** | **8** | **9** |
| Oral residue / dry feeling | **6** | **7** | **7** | **8** | **7** | **8** |
| Convergence | **7** | **7** | **7** | **7** | **7** | **7** |
| Throat irritation | **6** | **8** | **7** | **8** | **7** | **8** |
| Throat dryness | **7** | **8** | **7** | **8** | **7** | **8** |
| Nasal irritation | **7** | **7** | **7** | **7** | **7** | **7** |

Evaluation scores: 10 - no; 9 - micro; 8 - slightly; 7 - existence; 6: a little strong; ≤ 5 - strong

**Table 3: Comparison of the tar amount per cigarettes between the device of the present invention and the smoking ways in the prior art of the same cigarette**

| Cigarette sample No. | Smoking ways in the prior art (mg/cigarette) | The present invention (mg/cigarette) |
|---|---|---|
| 1 | **9.8** | **8.5** |
| 2 | **8.1** | **7.0** |
| 3 | **7.9** | **6.8** |
| 4 | **5.2** | **3.9** |
| 5 | **7.8** | **6.3** |
| 6 | **5.1** | **3.9** |

**Table 4: Comparison of the amount of free nicotine, total nicotine and the ratio of free nicotine / total nicotine produced by smoking the same cigarette between the device of the present invention and those by the smoking in the prior art.**

| Cigarette sample No. | Nicotine (mg/cigarette) | | Free Nicotine (mg/cigarette) | | Ratio of Free nicotine/total nicotine | |
|---|---|---|---|---|---|---|
| | The prior art | The present invention | The prior art | The present invention | The prior art | The present invention |
| 1 | **1.05** | **0.91** | **0.27** | **0.20** | **0.257** | **0.220** |
| 2 | **0.84** | **0.75** | **0.23** | **0.19** | **0.274** | **0.253** |
| 3 | **0.80** | **0.71** | **0.26** | **0.21** | **0.325** | **0.296** |
| 4 | **0.52** | **0.47** | **0.21** | **0.19** | **0.404** | **0.404** |
| 5 | **0.74** | **0.68** | **0.24** | **0.18** | **0.324** | **0.265** |
| 6 | **0.48** | **0.41** | **0.20** | **0.15** | **0.417** | **0.366** |

Referring to Table 1, the smoke amount of the same electrically-heating cigarette on the electrically-heating cigarette smoking device of the present invention is significantly increased.

Referring to Table 2, the sensory qualities (especially comfort feeling) of the same electrically-heating cigarette on the electrically-heating cigarette smoking device of the present invention are significantly improved.

Referring to Table 3, the tar content produced by smoking the same electrically-heating cigarette on the electrically-heating cigarette smoking device of the present invention is decreased.

Referring to Table 4, the free nicotine, the total nicotine and the ratio of free nicotine/nicotine produced by smoking the same electrically-heating cigarette on the electrically-heating cigarette smoking device of the present invention are all reduced to a certain extent, at the same time, the decrease in free nicotine exceeds the decrease in total nicotine, which plays an important role in improving sensory qualities (especially comfort feeling).

### Comparative experiment 2

Comparative Example 1: an electrically-heating cigarette smoking device having an electronic cigarette smoking function is different from the electrically-heating cigarette smoking device having an electronic cigarette smoking function in Example 1 only in that: in Comparative Example 1, the cigarette smoking device does not have a helical air flow passage, and air flow passages thereof are all straight type.

The same electrically-heating cigarette is smoked by using the electrically-heating cigarette smoking device having an electronic cigarette smoking function described in Embodiment 1 and Comparative Example 1, respectively, and the sensory quality was evaluated, the results of sensory quality evaluation are shown in Table 6.

### Sensory quality evaluation method:

Sensory quality evaluation items and scale settings are as follows: 5 items including irritation, saturation, dryness, smoking resistance and the smoke amount are set respectively, the maximum scale for each item is 20, each item is divided into the unit of 1 point, the meaning of each scale value corresponding to each evaluation item is shown in Table 5.

**Table 5**

| scopes | irritation | saturation | Dryness feeling | Smoking resistance | Smoke amount |
|---|---|---|---|---|---|
| 20 | Very small | Very good | Very light | moderate | Very big |
| 16 | Smaller | Better | Lighter | Rather moderate | Bigger |
| 12 | middle | moderate | moderate | Slight big | Slight small |
| 8 | larger | Worse | heavier | big | Small |
| 4 | very larger | Very bad | Very heavy | Very big | Very small |

Test procedure: samples to be tested and test table are provided to evaluation smoking experts to let them evaluate and smoke in accordance with the requirements of the table based on the indicators.

Statistic results: the evaluation and smoking results of all the evaluation and smoking persons are effective, the individual evaluation and smoking results are calculate to obtain the average values. The result is reserved for a decimal, the arithmetic mean values of the individual items are summed to get the total score, the results are retained in one decimal place and the evaluation results are shown in Table 6.

**Table 6**

| Samples | Irritation | saturation | Dryness feeling | Smoking resistance | Smoke amount | Total |
|---|---|---|---|---|---|---|
| Embodiment 1 | 18 | 17 | 19 | 18 | 19 | 91 |
| Comparative embodiment 1 | 15 | 14 | 16 | 17 | 18 | 80 |

As can be seen from Table 6, relative to Comparative Example 1, the irritation, the saturation and the drying feeling of Example 1 are significantly improved, and the dry feeling was improved too. The design of the helical air flow passage also has an important effect on the overall sensory quality of a smoking article.

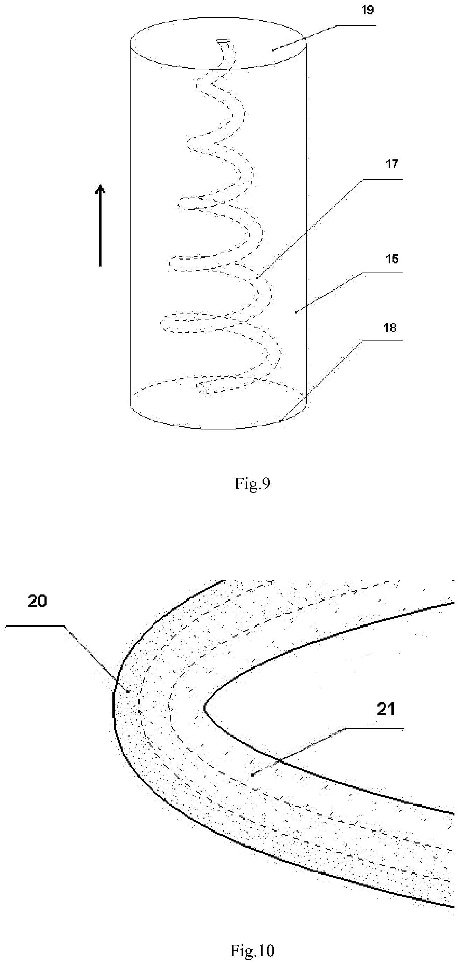

The effect drawing of the helical air flow passage during use is shown in Fig 10, due to the action of the centrifugal force, the aerosol particles in the smoke are enriched on the outside 20 of the air flow passage of the helical airflow passage, the aerosol particle concentration at the inner side 21 of the air flow passage is significantly lower than that on the outside of the air flow passage, it is surprisingly found that the saturation of the smoke will be increased by experiencing of this local enrichment effect. On the other hand, the a smoke transmission path of the helical air flow passage is designed to be longer than that of the traditional straight type air flow passage, which can make the smoke be better mixed, and reduce the irritation of the smoke.

## Claims

1. An electrically-heating cigarette smoking device having an electronic cigarette smoking function, comprising a cigarette holder (1 ), an electronically-vaporizing component and a mouth piece (15) which are mutually matched for assembly,
wherein the cigarette holder (1) comprises a cigarette holding cavity and an optional end cap; the electronically-vaporizing component comprises a shell (7) and the following assemblies located in the shell (7): a power supply (5), an air flow sensor switch (3) and an electronic vaporizer (8) having a central air flow passage; and the mouth piece (15) is provided with an air flow passage (16), the optional end cap is used for closing one open end of the shell (7) when present; the electrically-heating cigarette smoking device still comprises an air flow sensor switch gas inlet (2), the air flow sensor switch gas inlet (2) perforates the shell (7) and/or the optional end cap of the electrically-heating cigarette holder, and is in communication with the air flow passage (16) in the mouth piece (15) via the air flow sensor switch (3); **characterized in that** the cigarette holder (1) is an electrically-heating cigarette holder (1) having an electrical heater, **in that** the cigarette holding cavity is an electrically-heating cigarette holding cavity and **in that** the electronic vaporizer (8) and the electrically-heating cigarette holder (1) are arranged in parallel; the central air flow passage of the electronic vaporizer (8) and the electrically-heating cigarette holding cavity (1) are both in communication with the airflow passage (16) in the mouth piece (15).

2. The electrically-heating cigarette smoking device having an electronic cigarette smoking function according to claim 1 , **characterized in that** the electric heater is arranged in a way of circumferential heating, central heating, bottom heating or the combination thereof.

3. The electrically-heating cigarette smoking device having an electronic cigarette smoking function according to claim 1 , **characterized in that** part or all of the air flow passage (16) within the mouth piece (15) is a helical air flow passage (17) which comprises a connection end (18) at the downstream thereof and a suction end (19) at the upstream thereof.

4. The electrically-heating cigarette smoking device having an electronic cigarette smoking function according to claim 3, **characterized in that** the helical air flow passage (17) has an equal diameter wherein the diameter of the air flow passage of the helical air flow passage (17) is always equal from the connection end (18) to the suction end (19), or the helical air flow passage (17) has an varying diameter, wherein the diameter of the air flow passage of the helical air flow passage (17) is gradually changed in a continuous gradient from the big to the small or gradually changed in a continuous gradient from the small to the big.

5. The electrically-heating cigarette smoking device having an electronic cigarette smoking function according to claim 1 , **characterized in that** the electric vaporizer (8) comprises an electric heating wire, a liquid storing cavity for storing liquid to be vaporized, and a liquid guiding device for guiding the liquid to be vaporized to the electric heating wire.

6. The electrically-heating cigarette smoking device having an electronic cigarette smoking function according to claim 5, **characterized in that** the electric heating wire is located in the central air flow passage; the liquid guiding device is a liquid guiding cord or a liquid guiding pipe.

7. The electrically-heating cigarette smoking device having an electronic cigarette smoking function according to claim 1 , **characterized in that** the electrically-heating cigarette holding cavity of the electrically-heating cigarette holder (1) is provided therein with a variable-diameter holding device to hold the electrically-heating cigarettes with variable diameters, the variable-diameter holding device is selected from a plurality of overlapping washers which are successively reduced in diameter from an opening of the electrically-heating cigarette holding cavity, or a spring clamping head, or a multiple-jaw chuck holder.

8. The electrically-heating cigarette smoking device having an electronic cigarette smoking function according to claim 1, **characterized in that** the air flow sensor switch (3), the electronic vaporizer (8) and the electric heater are all electrically connected to the power supply (5), an electric connection way therebetween enables the air flow sensor switch (3) to control whether the electric vaporizer (8) and the electric heater are electrified or not.

9. Use of the electrically-heating cigarette smoking device having electronic cigarette smoking function according to any one of claims 1-8 for increasing smoke saturation and/or reducing laryngeal or nasal irritation for a smoker by nicotine in a smoke.

## Patentansprüche

1. Eine elektrisch heizende Zigarettenrauchvorrichtung mit einer elektronischen Zigarettenrauchfunktion, umfassend einen Zigarettenhalter (1), eine elektronisch verdampfende Komponente und ein Mundstück (15), die zur Montage aufeinander abgestimmt sind,
wobei der Zigarettenhalter (1) einen Zigarettenhaltehohlraum und eine optionale Endkappe umfasst; die elektronisch verdampfende Komponente umfasst eine Hülle (7) und die folgenden in der Hülle (7) angeordneten Einheiten: eine Stromversorgung (5), einen Luftstromsensorschalter (3) und einen elektronischen Verdampfer (8) mit einem zentralen Luftstromkanal; und das Mundstück (15) ist mit einem Luftstromkanal (16) versehen, die optionale Endkappe wird zum Verschließen eines offenen Endes der Hülle (7) verwendet, falls vorhanden; die elektrisch heizende Zigarettenrauchvorrichtung umfasst weiterhin einen Luftstromsensorschalter-Gaseinlass (2), wobei der Luftstromsensorschalter-Gaseinlass (2) die Hülle (7) und/oder die optionale Endkappe des elektrisch heizenden Zigarettenhalters perforiert und über den Luftstromsensorschalter (3) mit dem Luftstromkanal (16) im Mundstück (15) in Verbindung steht; **dadurch gekennzeichnet, dass** der Zigarettenhalter (1) ein elektrisch heizender Zigarettenhalter (1) mit einer elektrischen Heizvorrichtung ist, dass der Zigarettenhaltehohlraum ein elektrisch heizender Zigarettenhaltehohlraum ist und dass elektronischer Verdampfer (8) und der elektrisch heizende Zigarettenhalter (1) parallel angeordnet sind; der zentrale Luftstromkanal des elektronischen Verdampfers (8) und der elektrisch heizende Zigarettenhaltehohlraum (1) stehen beide mit dem Luftstromkanal (16) im Mundstück (15) in Verbindung.

2. Die elektrisch heizende Zigarettenrauchvorrichtung mit einer elektronischen Zigarettenrauchfunktion nach Anspruch 1, **dadurch gekennzeichnet, dass** die elektrische Heizvorrichtung als Umfangsheizung, Zentralheizung, Bodenheizung oder der Kombination aus diesen angeordnet ist.

3. Die elektrisch heizende Zigarettenrauchvorrichtung mit einer elektronischen Zigarettenrauchfunktion nach Anspruch 1, **dadurch gekennzeichnet, dass** der Luftstromkanal (16) innerhalb des Mundstücks (15) teilweise oder vollständig ein spiralförmiger Luftstromkanal (17) ist, der ein stromabwärts gelegenes Anschlussende (18) und ein stromaufwärts gelegenes Ansaugende (19) aufweist.

4. Die elektrisch heizende Zigarettenrauchvorrichtung mit einer elektronischen Zigarettenrauchfunktion nach Anspruch 3, **dadurch gekennzeichnet, dass** der spiralförmige Luftstromkanal (17) einen gleichen Durchmesser aufweist, wobei der Durchmesser des Luftstromkanals des spiralförmigen Luftstromkanals (17) vom Anschlussende (18) bis zum Ansaugende (19) immer gleich ist, oder der spiralförmige Luftstromkanal (17) einen variierenden Durchmesser hat, wobei der Durchmesser des Luftstromkanals des spiralförmigen Luftstromkanals (17) graduell in einem kontinuierlichen Gradienten vom Großen zum Kleinen verändert wird oder graduell in einem kontinuierlichen Gradienten vom Kleinen zum Großen verändert wird.

5. Die elektrisch heizende Zigarettenrauchvorrichtung mit einer elektronischen Zigarettenrauchfunktion nach Anspruch 1, **dadurch gekennzeichnet, dass** der elektrische Verdampfer (8) einen elektrischen Heizdraht, einen Flüssigkeitsspeicherhohlraum zum Speichern von zu verdampfender Flüssigkeit und eine Flüssigkeitsführungsvorrichtung zum Führen der zu verdampfenden Flüssigkeit zu dem elektrischen Heizdraht umfasst.

6. Die elektrisch heizende Zigarettenrauchvorrichtung mit einer elektronischen Zigarettenrauchfunktion nach Anspruch 5, **dadurch gekennzeichnet, dass** sich der elektrische Heizdraht im zentralen Luftstromkanal befindet; die Flüssigkeitsführungsvorrichtung ist eine Flüssigkeitsführungsschnur oder ein Flüssigkeitsführungsrohr.

7. Die elektrisch heizende Zigarettenrauchvorrichtung mit einer elektronischen Zigarettenrauchfunktion nach Anspruch 1, **dadurch gekennzeichnet, dass** der elektrisch heizende Zigarettenhaltehohlraum des elektrisch heizenden Zigarettenhalters (1) darin mit einer Haltevorrichtung für variable Durchmesser versehen ist, um die elektrisch heizenden Zigaretten mit variablen Durchmessern zu halten, wobei die Haltevorrichtung für variable Durchmesser ausgewählt ist aus einer Vielzahl überlappender Scheiben, die von einer Öffnung des elektrisch heizenden Zigarettenhaltehohlraums aus nacheinander im Durchmesser reduziert werden, oder einem Federklemmkopf oder einem Mehrbackenfutterhalter.

8. Die elektrisch heizende Zigarettenrauchvorrichtung mit einer elektronischen Zigarettenrauchfunktion nach Anspruch 1, **dadurch gekennzeichnet, dass** der Luftstromsensorschalter (3), der elektronische Verdampfer (8) und die elektrische Heizvorrichtung alle elektrisch mit der Stromversorgung (5) verbunden sind, ein elektrischer Verbindungsweg dazwischen ermöglicht es dem Luftstromsensorschalter (3), zu steuern, ob der elektrische Verdampfer (8) und die elektrische Heizvorrichtung elektrifiziert sind oder nicht.

9. Verwendung der elektrisch heizenden Zigarettenrauchvorrichtung mit elektronischer Zigarettenrauchfunktion gemäß einem der Ansprüche 1-8 zur Erhöhung der Rauchsättigung und/oder zur Verminderung von Kehlkopf- oder Nasenreizung für einen Raucher durch Nikotin im Rauch.

## Revendications

1. Dispositif de cigarette du type à chauffage électrique ayant une fonction de cigarette électronique, comprenant un porte-cigarette (1), un composant à vaporisation électronique et un embout (15) qui sont mutuellement adaptés pour l'assemblage,
dans lequel
le porte-cigarettes (1) comprend
une cavité de maintien de cigarette et un capuchon d'extrémité optionnel ; le composant de vaporisation électronique comprend une coque (7) et les ensembles suivants situés dans la coque (7) : une alimentation électrique (5), un commutateur de capteur d'écoulement d'air (3) et un vaporisateur électronique (8) ayant un passage d'écoulement d'air central ; et l'embout (15) est muni d'un passage d'écoulement d'air (16), le capuchon d'extrémité optionnel est utilisé pour fermer une extrémité ouverte de la coque (7) lorsqu'elle est présente ; le Dispositif de cigarette du type à chauffage électrique comprend en outre une entrée de gaz de commutation du capteur d'écoulement d'air (2), l'entrée de gaz de commutation du capteur d'écoulement d'air (2) perfore la coque (7) et/ou le capuchon d'extrémité optionnel du porte-cigarettes du type à chauffage électrique, et est en communication avec le passage d'écoulement d'air (16) dans l'embout (15) via le commutateur du capteur d'écoulement d'air (3) : **caractérisé en ce que** le porte-cigarette (1) est un porte-cigarette (1) du type à chauffage électrique ayant un dispositif de chauffage électrique, **en ce que** la cavité du porte-cigarette est une cavité du porte-cigarette du type à chauffage électrique et **en ce que** l'évaporateur électronique (8) et le porte-cigarette du type à chauffage électrique (1) sont disposés en parallèle ;
le passage d'écoulement d'air central de l'évaporateur électronique (8) et la cavité du porte-cigarette du type à chauffage électrique (1) sont tous deux en communication avec le passage d'écoulement d'air (16) dans l'embout (15).

2. Dispositif de cigarette du type à chauffage électrique ayant une fonction de cigarette électronique selon la revendication 1, **caractérisé en ce que** le dispositif de chauffage électrique est disposé de manière à chauffer la périphérie, le centre, le fond ou une combinaison de ceux-ci.

3. Dispositif de cigarette du type à chauffage électrique ayant une fonction de cigarette électronique selon la revendication 1, **caractérisé en ce qu'**une partie ou la totalité du passage d'écoulement d'air (16) à l'intérieur de l'embout (15) est un passage d'écoulement d'air hélicoïdal (17) qui comprend une extrémité de raccordement (18) en aval de celui-ci et une extrémité d'aspiration (19) en amont de celui-ci.

4. Dispositif de cigarette du type à chauffage électrique ayant une fonction de cigarette électronique selon la revendication 3, **caractérisé en ce que** le passage d'écoulement d'air hélicoïdal (17) a un diamètre égal, dans lequel le diamètre du passage d'écoulement d'air du passage d'écoulement d'air hélicoïdal (17) est toujours égal de l'extrémité de raccordement (18) à l'extrémité d'aspiration (19), ou le passage d'écoulement d'air hélicoïdal (17) a un diamètre variable, dans lequel le diamètre du passage d'écoulement d'air du passage d'écoulement d'air hélicoïdal (17) est progressivement modifié selon un gradient continu du grand au petit ou progressivement modifié selon un gradient continu du petit au grand.

5. Dispositif de cigarette du type à chauffage électrique ayant une fonction de cigarette électronique selon la revendication 1, **caractérisé en ce que** le vaporisateur électrique (8) comprend un fil chauffant électrique, une cavité de stockage de liquide pour stocker le liquide à vaporiser, et un dispositif de guidage de liquide pour guider le liquide à vaporiser vers le fil chauffant électrique.

6. Dispositif de cigarette du type à chauffage électrique ayant une fonction de cigarette électronique selon la revendication 5, **caractérisé en ce que** le fil chauffant électrique est situé dans le passage central d'écoulement d'air ; le dispositif de guidage de liquide est un cordon de guidage de liquide ou un tuyau de guidage de liquide.

7. Dispositif de cigarette du type à chauffage électrique ayant une fonction de cigarette électronique selon la revendication 1, **caractérisé en ce que** la cavité de maintien des cigarettes à chauffage électrique du porte-cigarettes à chauffage électrique (1) est pourvue d'un dispositif de maintien de diamètre variable pour maintenir les cigarettes à chauffage électrique de diamètre variable, le dispositif de maintien de diamètre variable est choisi parmi une pluralité de rondelles qui se chevauchent et dont le diamètre est successivement réduit à partir d'une ouverture de la cavité de maintien des cigarettes à chauffage électrique, ou une tête de serrage à ressort, ou un support de mandrin à mâchoires multiples.

8. Dispositif de cigarette du type à chauffage électrique ayant une fonction de cigarette électronique selon la revendication 1, **caractérisé en ce que** l'interrupteur du détecteur d'écoulement d'air (3), l'évaporateur électronique (8) et le dispositif de chauffage électrique sont tous reliés électriquement à l'alimentation électrique (5), une liaison électrique entre eux permet à l'interrupteur du détecteur d'écoulement d'air (3) de commander si l'évaporateur électrique (8) et le dispositif de chauffage électrique sont électrifiés ou non.

9. Dispositif de cigarette du type à chauffage électrique ayant une fonction de cigarette électronique selon l'une des revendications 1-8 pour augmenter la saturation de la fumée et/ou réduire l'irritation laryngée ou nasale pour un fumeur par la nicotine contenue dans une fumée.
